# EUROPÄISCHE PATENTSCHRIFT

(11) **EP 2 865 372 B1**
(45) Veröffentlichungstag und Bekanntmachung des Hinweises auf die Patenterteilung: **22.07.2020**
(21) Anmeldenummer: 13189795.1
(22) Anmeldetag: 22.10.2013
(51) Int. Cl.: A61K 9/48, A61K 9/50, A61K 9/68

(54) **Wirkstoffkapseln**
Capsules comprising actives
Capsules comprend actives

(43) Veröffentlichungstag der Anmeldung: 29.04.2015
(73) Patentinhaber: Symrise AG, 37603 Holzminden (DE)
(72) Erfinder: Weissbrodt, Jenny, 37603 Holzminden (DE); Aickele, Frank, 37603 Holzminden (DE); Bertram, Ralf, 37603 Holzminden (DE)
(74) Vertreter: Fabry, Bernd

(56) Entgegenhaltungen:
- EP-A1- 0 038 985
- WO-A1-02/064246
- GB-A- 1 483 542
- US-A- 4 376 113
- US-A1- 2004 151 778

## Beschreibung

### GEBIET DER ERFINDUNG

Die Erfindung befindet sich auf dem Gebiet der Nahrungsmitteltechnologie und betrifft neue wasserunlösliche Kapseln, die unter Verwendung von Proteinen, Polysacchariden, Cellulosederivaten und Tanninen erhalten werden und die darin enthaltenen Wirkstoffe verzögert freisetzen, ein Verfahren zur Herstellung der Kapseln sowie spezielle Verwendungen der Cellulosederivate und der Tannine im Rahmen des Herstellungsverfahrens.

### STAND DER TECHNIK

Für eine Vielzahl von Anwendungen, angefangen von der Pharmazie über die Kosmetik, Wasch- und Reinigungsmitteln bis hin zu Düngern hat die verzögerte Freisetzung von Wirkstoffen aus einer Kapselhülle in den letzten Jahren zunehmend an Bedeutung gewonnen. Auch im Nahrungsmittelbereich ist es vielfach wünschenswert, wenn insbesondere Geschmacksstoffe beim Einbringen in Wasser oder beim Kauen nicht spontan, sondern zeitverzögert freigesetzt werden ("controlled release").

Neben makroskopischen Produkten mit Durchmessern im Bereich bis hin zu 1 cm, sind insbesondere so genannte Mikrokapseln von Interesse. Hierunter werden vom Fachmann sphärische Aggregate mit einem Durchmesser im Bereich von etwa 0,0001 bis etwa 5 und vorzugsweise 0,005 bis 0,5 mm verstanden, die mindestens einen festen oder flüssigen Kern enthalten, der von mindestens einer kontinuierlichen Hülle umschlossen ist. Genauer gesagt handelt es sich um mit filmbildenden Polymeren umhüllte feindisperse flüssige oder feste Phasen, bei deren Herstellung sich die Polymere nach Emulgierung und Koazervation oder Grenzflächenpolymerisation auf dem einzuhüllenden Material niederschlagen.

Die Hülle solcher Mikrokapseln kann aus natürlichen, halbsynthetischen oder synthetischen Materialien bestehen.

Natürlich Hüllmaterialien sind beispielsweise Gummi Arabicum, Agar-Agar, Agarose, Maltodextrine, Alginsäure bzw. ihre Salze, z.B. Natrium- oder Calciumalginat, Fette und Fettsäuren, Cetylalkohol, Collagen, Chitosan, Lecithine, Gelatine, Albumin, Schellack, Polysaccharide, wie Stärke oder Dextran, Polypeptide, Proteinhydrolysate, Sucrose und Wachse.

Halbsynthetische Hüllmaterialien sind unter anderem chemisch modifizierte Cellulosen, insbesondere Celluloseester und -ether, z.B. Celluloseacetat, Ethylcellulose, Hydroxypropylcellulose, Hydroxypropylmethylcellulose und Carboxymethylcellulose, sowie Stärkederivate, insbesondere Stärkeether und -ester. Synthetische Hüllmaterialien sind beispielsweise Polymere wie Polyacrylate, Polyamide, Polyvinylalkohol oder Polyvinylpyrrolidon.

Die Verkapselung von Wirkstoffen unter Einsatz von Gelatine und Polysacchariden, speziell Gummi Arabicum ist Gegenstand zahlreicher Schutzrechte. Die ältesten Druckschriften stammen aus den Jahren 1958 bis 1974, nämlich US 3,041,288**;** JP 50 027826 A und JP 51 013387 A**.**

Aus der US 4,376,113 A (ROUSSEL UCLAF) ist ein Verfahren zur Herstellung von stabilen Suspensionen oder Pulver stabiler Mikrokapseln bekannt, bei dem man Mischungen aus Gelatine, Gummi Arabicum und einer Hydroxyethycellulose verkapselt und dann mit einer Mischung aus Glutaraldehyd und Tannin aushärtet.

Beispiele für Mikrokapseln des Stands der Technik sind folgende Handelsprodukte (in Klammern angegeben ist jeweils das Hüllmaterial) : *Hallcrest Microcapsules* (Gelatine, Gummi Arabicum), *Coletica Thalaspheres* (maritimes Collagen), *Lipotec Millicapseln* (Alginsäure, Agar-Agar), *Induchem Unispheres* (Lactose, mikrokristalline Cellulose, Hydroxypropy-Imethylcellulose); *Unicerin C30* (Lactose, mikrokristalline Cellulose, Hydroxypropylmethylcellulose), *Kobo Glycospheres* (modifizierte Stärke, Fettsäureester, Phospholipide), *Softspheres* (modifiziertes Agar-Agar) und *Kuhs Probiol Nanospheres* (Phospholipide) sowie *Primaspheres* und *Primasponges* (Chitosan, Alginate) und *Primasys* (Phospholipide).

Die klassischen Verkapselungsverfahren im Lebensmittelbereich sind jedoch alle wasserbasiert und liefern somit auch nur wasserlösliche Partikel. Da aber nahezu alle Lebensmittel Wasser enthalten, können die üblichen Technologien, wie z.B. Sprühtrocknung, Sprühgranulation oder Extrusion die geforderte Freisetzung bei Erhitzung des Lebensmittels oder beim Verzehr nicht oder nur sehr begrenzt leisten.

Somit hat eine erste Aufgabe der Erfindung darin bestanden, wasserunlösliches Partikel mit möglichst hoher Wirkstoffbeladung und einem D50-Wert von vorzugsweise etwa 10 bis etwa 150 µm zur Verfügung zu stellen, die den Wirkstoff erst bei der Nahrungszubereitung oder dem Verzehr freisetzen.

Ein weiterer Aspekt bei der Herstellung von Mikrokapseln besteht darin, dass die Produkte unmittelbar nach ihrer Herstellung keine ausreichende mechanische Festigkeit besitzen, sondern leicht aufbrechen. Zu diesem Zweck werden die Kapseln einer Härtung unterzogen, sei es durch Bildung wasserunlöslicher Calciumsalze auf der Hülle oder durch Vernetzungsreaktionen mit Aldehyden, wie beispielsweise Formaldehyd oder Glutaraldehyd. Die Verwendung solcher Vernetzer ist jedoch aus toxikologischen Gründen unerwünscht, so dass eine weitere Teilaufgabe der Erfindung darin bestanden hat, zusätzlich auch noch Mikrokapseln der geschilderten Art zu entwickeln, die ein alternatives Vernetzungsmittel enthalten, das lebensmittelrechtlich zulässig und aus technischer Sicht hinreichend wirksam ist.

Ein dritter Aspekt der Erfindung ist auf den Umstand gerichtet, dass die Herstellung von Mikrokapseln über den Weg der Koazervation, Härtung und gegebenenfalls Trocknung ausgesprochen zeitaufwendig ist und große Mengen Wasser zur Dispergierung erfordert. Wünschenswert ist es hingegen, die Wassermengen zu verringern und insbesondere die Herstellzeit erheblich zu verkürzen.

### BESCHREIBUNG DER ERFINDUNG

Eine erster Gegenstand der Erfindung betrifft Wirkstoffkapseln, die dadurch erhältlich sind, dass man unter Eintrag von mechanischer und thermischer Energie
(a) Proteine, Polysaccharide und Cellulosederivate in Wasser löst bzw. dispergiert,
(b) die zu verkapselnden Wirkstoffe zugibt,
(c) die so erhaltenen Koazervate unter Zugabe von Tanninen aushärtet und gegebenenfalls
(d) anschließend einer Sprühtrocknung unterwirft,
mit der Maßgabe, dass die Kapseln frei von Formaldehyd und Glutaraldehyd sind.

Ein zweiter Aspekt der Erfindung ist auf ein Verfahren zur Herstellung von Wirkstoffkapseln, gerichtet, bei dem man unter Eintrag von mechanischer und thermischer Energie
(a) Proteine, Polysaccharide und Cellulosederivate in Wasser löst bzw. dispergiert,
(b) die zu verkapselnden Wirkstoffe zugibt,
(c) die so erhaltenen Koazervate unter Zugabe von Tanninen aushärtet und gegebenenfalls
(d) anschließend einer Sprühtrocknung unterwirft,
mit der Maßgabe, dass die Kapseln frei von Formaldehyd und Glutaraldehyd sind.

Überraschenderweise wurde gefunden, dass mit Hilfe des erfindungsgemäßen Verfahrens wasserunlösliche Koazervate zur Verfügung gestellt werden können, die einen D50-Wert von insbesondere etwa 10 bis etwa 150 µm, speziell etwa 50 bis etwa 100 µm aufweisen und sich problemlos durch Sprühtrocknung von Wasser befreien lassen. Die Koazervate können mit den unterschiedlichsten Wirkstoffen in hohen Mengen beladen werden und setzen diese in wässrigem Medium erst nach Stunden in nennenswerten Mengen frei, weshalb sie sich für bestimmte Anwendungen - wie beispielsweise im Cateringbereich - besonders geeignet sind.

Die Partikelgröße kann gemäß der Anforderungen an das Zielprodukt und die Freisetzung im weiten Grenzen variiert werden. Ist eine schnellere Freisetzung gewünscht, können die Partikel durch Variation der Verfahrensbedingungen Rührgeschwindigkeit und Temperatur größer (D50 von etwa 100 µm) oder für eine längere Freisetzungszeit kleiner (D50 <50 µm) hergestellt werden.

Das erfindungsgemäße Verfahren kommt zudem mit einer deutlich geringeren Menge Wasser aus und erlaubt, die Kapseln - einschließlich Sprühtrocknung - innerhalb von maximal 12 Stunden herzustellen; konventionelle Prozesse benötigen üblicherweise die doppelte Zeit.

Mit Tannin wurde ein alternatives Härtungs- bzw. Vernetzungsmittel gefunden, das nicht nur für den Lebensmittelbereich zugelassen ist, sondern den Kapselhüllen eine Bruchfestigkeit verleiht, wie man es sonst nur von der Behandlung mit Formaldehyd oder Glutaraldehyd kennt.

### HÜLLSUBSTANZEN

Bei den Wirkstoffkapseln handelt es sich um echte Hülle-Kern-Kapseln, die durch Koazervation der Hüllstoffe entstehen und dabei den Wirkstoff einschließen. Die Hülle wird von den drei Komponenten (a1), (a2) und (a3) bilden, wobei es im eigentlichen Sinne nur zu einer Koazervation der Proteine mit den Polysacchariden kommt, während die Cellulosederivate als Schutzkolloide fungieren, die das Verkleben der Kapseln verhindern. Dies ist für sich ebenfalls eine überraschende Erkenntnis, da Cellulosederivate ansonsten eher zum "verkleben" eingesetzt werden. Durch die Verwendung der Cellulosederivate kann gleichzeitig die Menge an Wasser - über die sonst die Agglomeration verhindert wird - halbiert werden.

**Proteine.** Als Proteine (Komponente a1) können Gelatinen unterschiedlichster Herkunft eingesetzt werden, also beispielsweise auf Basis Rind, Schwein, Geflügel oder Fisch. Vorzugsweise besitzen sie dabei eine Gelfestigkeit im Bereich von etwa 100 bis etwa 300 Bloom, vorzugsweise etwa 150 bis etwa 290 Bloom und insbesondere etwa 220 bis 260 Bloom¹.
¹Die Kennzahl ist dabei die Masse in Gramm, die benötigt wird, damit ein Stempel von 0,5 Zoll Durchmesser die Oberfläche einer 6,67 % Gelatine/Wasser-Mischung vier Millimeter tief verformt, ohne sie zu zerreißen. Der Versuch findet standardisiert bei exakt 10 °C mit einer vorhergehenden Alterung der Gelatine von 17 Stunden statt.

**Polysaccharide.** Als Hüllsubstanzen vom Typ der Polysaccharide einschließlich der Heteropolysaccharide (Komponente a2) kommen beispielsweise Pektine, Xanthane (auch Xanthan Gum) sowie deren Mischungen in Frage. Vorzugsweise wird als Hüllsubstanz Gummi arabicum eingesetzt, beispielsweise vom Typ *Acacia seyal.* Gummi arabicum besteht aus farblosen bis braunen, matten, spröden, geruchlosen Stücken mit glänzendem Bruch, die sich in warmem Wasser zu einer klaren, zähen, klebrigen, fad schmeckenden und schwach sauer reagierenden Flüssigkeit auflösen. Es setzt sich hauptsächlich aus den sauren Erdalkali- und Alkalisalzen der Arabinsäure (Polyarabinsäure) zusammen, unter der man ein verzweigtes, aus L-Arabinose, D-Galactose, L-Rhamnose und D-Glucuronsäure im Verhältnis 3:3:1:1 bestehendes Polysaccharid versteht.

Auch Agarosen kommen als Hüllsubstanzen in Betracht, welche in Form des aus Rotalgen zu gewinnenden Agar-Agar auch zusammen mit bis zu 30 Gew.-% nicht-gelbildenden Agaropektinen vorliegen können. Hauptbestandteil der Agarosen sind lineare Polysaccharide aus D-Galaktose und 3,6-Anhydro-L-galaktose, die alternierend β-1,3- und β-1,4-glykosidisch verknüpft sind.

Die Polysaccharide besitzen vorzugsweise ein Molekulargewicht im Bereich von 110.000 bis 160.000 und sind sowohl farb- als auch geschmacklos. Es sind weiterhin solche Typen bevorzugt, die noch in 1-Gew.-%iger wässriger Lösung Gele bilden, die nicht unterhalb von 80 °C schmelzen und sich bereits oberhalb von 40 °C wieder verfestigen.

**Cellulosederivate.** Als Cellulosederivate (Komponente a3) kommen neben chemisch nicht modifizierter Cellulose insbesondere Celluloseether und Celluloseester mit Substitutionsgraden oberhalb von 80. Vorzugsweise oberhalb von 90 und insbesondere oberhalb von 99 % in Betracht. Besonders bevorzugt sind Carboxymethylcellulosen, die nach E466 als Lebensmittelzusatzstoff zugelassen sind, wie beispielsweise die Type Ceroga 4510 C (Brenntag).

### WIRKSTOFFE

Die Auswahl der Wirkstoffe, die es zu verkapseln gilt, ist unkritisch und richtet sich im Wesentlichen nur danach, welcher Anwendungszweck verfolgt werden soll. Es kann sich dabei z.B. um Farbstoffe handeln, da die Erfindung jedoch in erster Linie auf Nahrungsmittel abzielt, handelt es sich bei den Wirkstoffen vorzugsweise um Aromen, insbesondere solche Stoffe, die hinreichen lipophil und wasserunlöslich sind und sich daher in wässrigem Milieu auch leicht verkapseln lassen.

Typische Beispiele für Aromen umfassen: Acetophenon, Allylcapronat, alpha-lonon, beta-lonon, Anisaldehyd, Anisylacetat, Anisylformiat, Benzaldehyd, Benzothiazol, Benzylacetat, Benzylalkohol, Benzylbenzoat, beta-lonon, Butylbutyrat, Butylcapronat, Butylidenphthalid, Carvon, Camphen, Caryophyllen, Cineol, Cinnamylacetat, Citral, Citronellol, Citronellal, Citronellylacetat, Cyclohexylacetat, Cymol, Damascon, Decalacton, Dihydrocumarin, Dimethylanthranilat, Dimethylanthranilat, Dodecalacton, Ethoxyethylacetat, Ethylbuttersäure, Ethylbutyrat, Ethylcaprinat, Ethylcapronat, Ethylcrotonat, Ethylfuraneol, Ethylguajakol, Ethylisobutyrat, Ethylisovalerianat, Ethyllactat, Ethylmethylbutyrat, Ethylpropionat, Eucalyptol, Eugenol, Ethylheptylat, 4-(p-Hydroxyphenyl)-2-butanon, gamma-Decalacton, Geraniol, Geranylacetat, Geranylacetat, Grapefruitaldehyd, Methyldihydrojasmonat (z.B. Hedion®), Heliotropin, 2-Heptanon, 3-Heptanon, 4-Heptanon, trans-2-Heptenal, cis-4-Heptenal, trans-2-Hexenal, cis-3-Hexenol, trans-2-Hexensäure, trans-3-Hexensäure, cis-2-Hexenylacetat, cis-3-Hexenylacetat, cis-3-Hexenylcapronat, trans-2-Hexenylcapronat, cis-3-Hexenylformiat, cis-2-Hexylacetat, cis-3-Hexylacetat, trans-2-Hexylacetat, cis-3-Hexylformiat, para-Hydroxybenzylaceton, Isoamylalkohol, Isoamylisovalerianat, Isobutylbutyrat, Isobutyraldehyd, Isoeugenolmethylether, Isopropylmethylthiazol, Laurinsäure, Leavulinsäure, Linalool, Linalooloxid, Linalylacetat, Menthol, Menthofuran, Methylanthranilat, Methylbutanol, Methylbuttersäure, 2-Methylbutylacetat, Methylcapronat, Methylcinnamat, 5-Methylfurfural, 3,2,2-Methylcyclopentenolon, 6,5,2-Methylheptenon, Methyldihydrojasmonat, Methyljasmonat, 2-Methylmethylbutyrat, 2-Methyl-2-Pentenolsäure, Methylthiobutyrat, 3,1-Methylthiohexanol, 3-Methylthiohexylacetat, Nerol, Nerylacetat, trans,trans-2,4-Nonadienal, 2,4-Nonadienol, 2,6-Nonadienol, 2,4-Nonadienol, Nootkaton, delta Octalacton, gamma Octalacton, 2-Octanol, 3-Octanol, 1,3-Octenol, 1-Octylacetat, 3-Octylacetat, Palmitinsäure, Paraldehyd, Phellandren, Pentandion, Phenylethylacetat, Phenylethylalkohol, Phenylethylalkohol, Phenylethylisovalerianat, Piperonal, Propionaldehyd, Propylbutyrat, Pulegon, Pulegol, Sinensal, Sulfurol, Terpinen, Terpineol, Terpinolen, 8,3-Thiomenthanon, 4,4,2-Thiomethylpentanon, Thymol, delta-Undecalacton, gamma-Undecalacton, Valencen, Valeriansäure, Vanillin, Acetoin, Ethylvanillin, Ethylvanillinisobutyrat (= 3-Ethoxy-4-isobutyryloxybenzaldehyd), 2,5-Dimethyl-4-hydroxy-3(2H)-furanon und dessen Abkömmlinge (dabei vorzugsweise Homofuraneol (= 2-Ethyl-4-hydroxy-5-methyl-3(2H)-furanon), Homofuronol (= 2-Ethyl-5-methyl-4-hydroxy-3(2H)-furanon und 5-Ethyl-2-methyl-4-hydroxy-3(2H)-furanon), Maltol und Maltol-Abkömmlinge (dabei vorzugsweise Ethylmaltol), Cumarin und Cumarin-Abkömmlinge, gamma-Lactone (dabei vorzugsweise gamma-Undecalacton, gamma-Nonalacton, gamma-Decalacton), delta-Lactone (dabei vorzugsweise 4-Methyldeltadecalacton, Massoilacton, Deltadecalacton, Tuberolacton), Methylsorbat, Divanillin, 4-Hydroxy-2(oder 5)-ethyl-5(oder 2)-methyl-3(2H)furanon, 2-Hydroxy-3-methyl-2-cyclopentenon, 3-Hydroxy-4,5-dimethyl-2(5H)-furanon, Essigsäureisoamylester, Buttersäureethylester, Buttersäure-n-butylester, Buttersäureisoamylester, 3-Methylbuttersäureethylester, n-Hexansäureethylester, n-Hexansäureallylester, n-Hexansäure-n-butylester, n-Octansäureethylester, Ethyl-3-methyl-3-phenylglycidat, Ethyl-2-trans-4-cis-decadienoat, 4-(p-Hydroxyphenyl)-2-butanon, 1,1-Dimethoxy-2,2,5-trimethyl-4-hexan, 2,6-Dimethyl-5-hepten-1-al und Phenylacetaldehyd, 2-Methyl-3-(methylthio)furan, 2-Methyl-3-furanthiol, bis(2-Methyl-3-furyl)disulfid, Furfurylmercaptan, Methional, 2-Acetyl-2-thiazolin, 3-Mercapto-2-pentanon, 2,5-Dimethyl-3-furanthiol, 2,4,5-Trimethylthiazol, 2-Acetylthiazol, 2,4-Dimethyl-5-ethylthiazol, 2-Acetyl-1-pyrrolin, 2-Methyl-3-ethylpyrazin, 2-Ethyl-3,5-dimethylpyrazin, 2-Ethyl-3,6-dimethylpyrazin, 2,3-Diethyl-5-methylpyrazin, 3-Isopropyl-2-methoxypyrazin, 3-Isobutyl-2-methoxypyrazin, 2-Acetylpyrazin, 2-Pentylpyridin, (E,E)-2,4-Decadienal, (E,E)-2,4-Nonadienal, (E)-2-Octenal, (E)-2-Nonenal, 2-Undecenal, 12-Methyltridecanal, 1-Penten-3-on, 4-Hydroxy-2,5-dimethyl-3(2H)-furanon, Guajakol, 3-Hydroxy-4,5-dimethyl-2(5H)-furanon, 3-Hydroxy-4-methyl-5-ethyl-2(5H)-furanon, Zimtaldehyd, Zimtalkohol, Methylsalicylat, Isopulegol sowie (hier nicht explizit genannte) Stereoisomere, Enantiomere, Stellungsisomere, Diastereomere, cis/trans-Isomere bzw. Epimere dieser Substanzen.

Für Anwendungen, bei denen die Kapseln nicht konsumiert werden, jedoch mit der Mundschleimhaut in Kontakt kommen, können auch Parfümöle verkapselt werden.

Als Parfümöle seien genannt Gemische aus natürlichen und synthetischen Riechstoffen. Natürliche Riechstoffe sind Extrakte von Blüten (Lilie, Lavendel, Rosen, Jasmin, Neroli, Ylang-Ylang), Stengeln und Blättern (Geranium, Patchouli, Petitgrain), Früchten (Anis, Koriander, Kümmel, Wacholder), Fruchtschalen (Bergamotte, Zitrone, Orangen), Wurzeln (Macis, Angelica, Sellerie, Kardamon, Costus, Iris, Calmus), Hölzern (Pinien-, Sandel-, Guajak-, Zedern-, Rosenholz), Kräutern und Gräsern (Estragon, Lemongras, Salbei, Thymian), Nadeln und Zweigen (Fichte, Tanne, Kiefer, Latschen), Harzen und Balsamen (Galbanum, Elemi, Benzoe, Myrrhe, Olibanum, Opoponax). Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind z.B. Benzylacetat, Phenoxyethylisobutyrat, p-tert.-Butylcyclohexylacetat, Linalylacetat, Dimethylbenzylcarbinylacetat, Phenylethylacetat, Linalylbenzoat, Benzylformiat, Ethylmethylphenylglycinat, Allylcyclohexylpropionat, Styrallylpropionat und Benzylsalicylat. Zu den Ethern zählen beispielsweise Benzylethylether, zu den Aldehyden z.B. die linearen Alkanale mit 8 bis 18 Kohlenstoffatomen, Citral, Citronellal, Citronellyloxyacetaldehyd, Cyclamenaldehyd, Hydroxycitronellal, Lilial und Bourgeonal, zu den Ketonen z.B. die Jonone, α-Isomethylionon und Methylcedrylketon, zu den Alkoholen Anethol, Citronellol, Eugenol, Isoeugenol, Geraniol, Linalool, Phenylethylalkohol und Terpineol, zu den Kohlenwasserstoffen gehören hauptsächlich die Terpene und Balsame. Bevorzugt werden jedoch Mischungen verschiedener Riechstoffe verwendet, die gemeinsam eine ansprechende Duftnote erzeugen. Auch ätherische Öle geringerer Flüchtigkeit, die meist als Aromakomponenten verwendet werden, eignen sich als Parfümöle, z.B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl, Labolanumöl und Lavandinöl. Vorzugsweise werden Bergamotteöl, Dihydromyrcenol, Lilial, Lyral, Citronellol, Phenylethylalkohol, α-Hexylzimtaldehyd, Geraniol, Benzylaceton, Cyclamenaldehyd, Linalool, Boisambrene Forte, Ambroxan, Indol, Hedione, Sandelice, Citronenöl, Mandarinenöl, Orangenöl, Allylamylglycolat, Cyclovertal, Lavandinöl, Muskateller Salbeiöl, β-Damascone, Geraniumöl Bourbon, Cyclohexylsalicylat, Vertofix Coeur, Iso-E-Super, Fixolide NP, Evernyl, Iraldein gamma, Phenylessigsäure, Geranylacetat, Benzylacetat, Rosenoxid, Romilllat, Irotyl und Floramat allein oder in Mischungen, eingesetzt.

Als Aromen kommen beispielsweise Pfefferminzöl, Krauseminzöl, Anisöl, Sternanisöl, Kümmelöl, Eukalyptusöl, Fenchelöl, Citronenöl, Wintergrünöl, Nelkenöl, Menthol und dergleichen in Frage.

### KOAZERVATION

Bei der Verkapselungsreaktion handelt es sich um eine Koazervation zwischen den Proteinen und den Polysacchariden, vorzugsweise von Gelatine und Gummi arabicum, bei dem das Cellulosederivat lediglich als Schutzkolloid dient und die Agglomeration der frisch gebildeten Kapseln unterbindet.

Vorzugsweise setzt man die Komponenten (a1), (b1) und (c1) im Gewichtsverhältnis von etwa 10:(1 bis 5):(0,5 bis 2) und insbesondere 10:(2 bis 4):(1 bis 1,5) ein. Die Koazervation erfolgt unter Eintragung von mechanischer und thermischer Energie, d.h. während der Herstellung wird die Reaktionsmischung gerührt und auf eine Temperatur im Bereich von etwa 10 bis etwa 90 °C eingestellt. Dabei hat es sich als vorteilhaft erwiesen, die Koazervation der Hüllsubstanzen bei Temperaturen im Bereich von etwa 50 bis etwa 90 bei schwacher Rührleistung zu beginnen und dann die Wirkstoffe beizugeben, was automatisch zu einer Abkühlung des Reaktionsansatzes auf etwa 40 °C führt. Jetzt empfiehlt es sich, die Rührleistung zu steigern und die Mischung schrittweise bis auf etwa 10 °C abzukühlen. Dabei gilt, dass mit stärkerer Rührleistung und längerer Reaktionszeit kleinere Kapseln gebildet werden und umgekehrt. Als weitere Zusatzstoffe kommen hier physiologisch verträgliche Säuren, wie Essigsäure, Zitronensäure oder Milchsäure in Betracht, mit denen der pH-Wert der Lösung im sauren Bereich bei etwa 3 bis 5 gehalten wird. Da die Ansätze dazu neigen, Schaum zu entwickeln, ist es ebenfalls empfehlenswert Entschäumer, beispielsweise handelsübliche Siliconentschäumer beizugeben.

### HÄRTUNG BZW: VERNETZUNG

Nach der Koazervation weisen die Kapseln noch eine flexible Hülle auf, die keine sonderliche Stabilität besitzt und daher leicht aufbricht. Zu diesem Zweck wird eine Härtung bzw. Vernetzung der Hülle durchgeführt. Für diesen Zweck werden natürliche pflanzliche Gerbstoffe vom Typ der Tannine eingesetzt, bei denen es sich chemisch betrachtet um Proanthocyanidine handelt, wie sie in dikotylen Stauden, Sträuchern und Blättern besonders in den Tropen und Subtropen zu finden sind. Die Terpene weisen in der Regel molekulare Gewichte im Bereich von 500 bis 3.000 KDa auf.

Ein bevorzugtes Beispiel für ein geeignetes Tannin ist das Corigallin, ein verbrücktes 1,3,5-Trigalloyltannin der folgenden Formel:

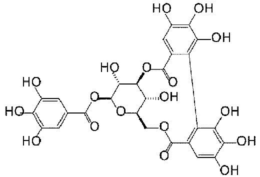

Tannine werden danach klassifiziert, ob sie hydrolysierbar sind oder nicht. Erstere können zu Glucose, andere mehrwertige Alkohole, Gallussäure oder Ellagsäure abgebaut werden. Kondensierte Tannine bestehen hingegen aus miteinander polymerisierten flavonoiden Phenolen, wie Catechin, Epicatechin oder Anthocyanen.

Zur Härtung wird eine wässerige Zubereitung der Tannine der die rohen Kapseln enthaltenden wässrigen Dispersion zugegeben und bei etwa 10 bis etwa 20 °C über etwa 5 bis etwa 10 h gerührt. Üblicherweise setzt man die Tannine in Mengen von etwa 0,1 bis etwa 2 Gew.-% und vorzugsweise von etwa 0,5 bis etwa 1,5 Gew.-% - bezogen auf die Koazervate - zu.

### SPRÜHTROCKNUNG

Nach der Koazervation und Härtung liegen die erfindungsgemäßen Kapseln als Dispersion in Wasser vor. In dieser Form sind sie grundsätzlich bereits verkaufsfähig, zu Konservierungszwecken empfiehlt es sich jedoch, sie zu trocknen. Grundsätzlich kommen dafür auf Verfahren wie die Lyophilisierung in Frage, bevorzugt ist jedoch eine Sprühtrocknung beispielsweise in der Wirbelschicht. Dabei hat es sich als vorteilhaft erwiesen, der Dispersion bei Temperaturen von etwa 20 bis etwa 50 °C und vorzugsweise etwa 40 °C weitere Polysaccharide, vorzugsweise Dextrine und insbesondere Maltodextrine zuzugeben, die den Trockenprozess unterstützen und die Kapseln während dieses Vorgangs schützen. Dabei kann die Einsatzmenge der Polysaccharide etwa 50 bis etwa 150 Gew.-% und vorzugsweise etwa 80 bis etwa 120 Gew.-% bezogen auf die Kapselmasse in der Dispersion betragen.

Die Sprühtrocknung selbst kann in konventionellen Sprühanlagen kontinuierlich oder batchweise durchgeführt werden, wobei die Einlasstemperatur bei etwa 170 bis etwa 200 °C und vorzugsweise etwa 180 bis 185 °C liegt und die Austrittstemperatur etwa 70 bis etwa 80 °C und vorzugsweise etwa 72 bis 78 °C beträgt.

### GEWERBLICHE ANWENDBARKEIT

Ein weiterer Gegenstand der vorliegenden Patentanmeldung ist auf Zubereitungen zur oralen Aufnahme gerichtet, die die erfindungsgemäßen Wirkstoffkapseln enthalten, beispielsweise in Mengen von 0,1 bis etwa 10 Gew.-%, vorzugsweise etwa 1 bis etwa 8 Gew.-% und insbesondere etwa 2 bis etwa 5 Gew.-%

Unter Zubereitungen zur oralen Aufnahme sind in erster Linie Nahrungs- und Genussmittel zu verstehen. Ebenfalls umfasst werden aber auch solche Zubereitungen, die zwar mit der Mundschleimhaut in Kontakt kommen, jedoch nicht zum Verzehr gedacht sind. Dazu gehören Mund- und Zahnpflegemittel und Kaugummis.

### NAHRUNGSMITTEL

Vorzugsweise handelt es sich bei den Nahrungsmitteln um Backwaren, beispielsweise Brot, Trockenkekse, Kuchen, sonstiges Gebäck, Süßwaren (beispielsweise Schokoladen, Schokoladenriegelprodukte, sonstige Riegelprodukte, Fruchtgummi, Hart- und Weichkaramellen, Kaugummi), alkoholische oder nicht-alkoholische Getränke (beispielsweise Kaffee, Tee, Eistee, Wein, weinhaltige Getränke, Bier, bierhaltige Getränke, Liköre, Schnäpse, Weinbrände, (carbonisierte) fruchthaltige Limonaden, (carbonisierte) isotonische Getränke, (carbonisierte) Erfrischungsgetränke, Nektare, Schorlen, Obst- und Gemüsesäfte, Frucht- oder Gemüsesaftzubereitungen, Instantgetränke (beispielsweise Instant-Kakao-Getränke, Instant-Tee-Getränke, Instant-Kaffeegetränke, Instant-Fruchtgetränke), Fleischprodukte (beispielsweise Schinken, Frischwurst- oder Rohwurstzubereitungen, gewürzte oder marinierte Frisch- oder Pökelfleischprodukte), Eier oder Eiprodukte (Trockenei, Eiweiß, Eigelb), Getreideprodukte (beispielsweise Frühstückscerealien, Müsliriegel, vorgegarte Fertigreis-Produkte), Milchprodukte (beispielsweise Milchgetränke, Buttermilchgetränke, Milcheis, Joghurt, Kefir, Frischkäse, Weichkäse, Hartkäse, Trockenmilchpulver, Molke, Molkegetränke, Butter, Buttermilch, teilweise oder ganz hydrolisierte Milchprotein-haltige Produkte), Produkte aus Sojaprotein oder anderen Sojabohnen-Fraktionen (beispielsweise Sojamilch und daraus gefertigte Produkte, Fruchtgetränke mit Sojaprotein, Sojalecithin-haltige Zubereitungen, fermentierte Produkte wie Tofu oder Tempe oder daraus gefertigte Produkte), Produkte aus anderen pflanzlichen Proteinquellen, beispielsweise Haferprotein-Getränke, Fruchtzubereitungen (beispielsweise Konfitüren, Fruchteis, Fruchtsoßen, Fruchtfüllungen), Gemüsezubereitungen (beispielsweise Ketchup, Soßen, Trockengemüse, Tiefkühlgemüse, vorgegarte Gemüse, eingekochte Gemüse), Knabberartikel (beispielsweise gebackene oder frittierte Kartoffelchips oder Kartoffelteigprodukte, Extrudate auf Mais- oder Erdnussbasis), Produkte auf Fett- und Ölbasis oder Emulsionen derselben (beispielsweise Mayonnaise, Remoulade, Dressings), sonstige Fertiggerichte und Suppen (beispielsweise Trockensuppen, Instant-Suppen, vorgegarte Suppen), Gewürze, Würzmischungen sowie insbesondere Aufstreuwürzungen (englisch: Seasonings), die beispielsweise im Snackbereich Anwendung finden.

Diese Nahrungsmittel können weitere Inhaltsstoffe aufweisen, wie beispielsweise: **Süßstoffe.** Als Süßstoffe oder süß schmeckende Zusatzstoffe kommen zunächst Kohlenhydrate und speziell Zucker in Frage, wie etwa Sucrose/Saccharose, Trehalose, Lactose, Maltose, Melizitose, Raffinose, Palatinose, Lactulose, D-Fructose, D-Glucose, D-Galactose, L-Rhamnose, D-Sorbose, D-Mannose, D-Tagatose, D-Arabinose, L-Arabinose, D-Ribose, D-Glycerinaldehyd, oder Maltodextrin. Ebenfalls geeignet sind pflanzliche Zubereitungen, die diese Stoffe enthalten, beispielsweise auf Basis von Zuckerrüben (Beta vulgaris ssp., Zuckerfraktionen, Zuckersirup, Melasse), Zuckerrohr (Saccharum officinarum ssp., Melasse, Zuckerrohrsirup), Ahornsirup (Acer ssp.) oder Agaven (Agavendicksaft).

In Betracht kommen auch
- synthetische, d.h. in der Regel enzymatisch hergestellte Stärke oder Zuckerhydrolysate (Invertzucker, Fructosesirup);
- Fruchtkonzentrate (z.B. auf Basis von Äpfeln oder Birnen);
- Zuckeralkohole (z.B. Erythritol, Threitol, Arabitol, Ribotol, Xylitol, Sorbitol, Mannitol, Dulcitol, Lactitol);
- Proteine (z.B. Miraculin, Monellin, Thaumatin, Curculin, Brazzein);
- Süßstoffe (z.B. Magap, Natriumcyclamat, Acesulfam K, Neohesperidin Dihydrochalcon, Saccharin Natriumsalz, Aspartam, Superaspartam, Neotam, Alitam, Sucralose, Stevioside, Rebaudioside, Lugduname, Carrelame, Sucrononate, Sucrooctate, Monatin, Phenylodulcin);
- Süß schmeckende Aminosäuren (z.B. Glycin, D-Leucin, D-Threonin, D-Asparagin, D-Phenylalanin, D-tryptophn, L-Prolin);
- Weitere süß schmeckende niedermolekulare Substanzen, wie z.B. Hernandulcin, Dihydrochalconglykoside, Glycyrrhizin, Glycerrhetinsäure, ihre Derivate und Salze, Extrakte von Lakritz (Glycyrrhizza glabra ssp.), Lippia dulcis Extrakte, Momordica ssp. Extrakte oder
- Einzelsubstanzen wie z.B. Momordica grosvenori [Luo Han Guo] und die daraus gewonnenen Mogroside, Hydrangea dulcis oder Stevia ssp. (z.B. Stevia rebaudiana) Extrakte.

**Lebensmittelsäuren.** Die Nahrungsmittel können Carbonsäuren enthalten. Säuren im Sinne der Erfindung sind bevorzugt in Lebensmitteln zulässige Säuren sowie deren Salze, insbesondere die hier genannten:
- E 260: - Essigsäure
- E 270: - Milchsäure
- E 290: - Kohlendioxid
- E 296: - Apfelsäure
- E 297: - Fumarsäure
- E 330: - Citronensäure
- E 331: - Natriumcitrat
- E 332: - Kaliumcitrat
- E 333: - Calciumcitrat
- E 334: - Weinsäure
- E 335: - Natriumtartrat
- E 336: - Kaliumtartrat
- E 337: - Natrium-Kaliumtartrat
- E 338: - Phosphorsäure
- E 353: - Metaweinsäure
- E 354: - Calciumtartrat
- E 355: - Adipinsäure
- E 363: - Bernsteinsäure
- E 380: - Triammoniumcitrat
- E 513: - Schwefelsäure
- E 574: - Gluconsäure
- E 575: - Glucono-delta-Lacton

**Säureregulatoren.** Säureregulatoren sind Lebensmittelzusatzstoffe, die den Säuregrad oder die Basizität und damit den gewünschten pH-Wert eines Lebensmittels konstant halten. Es handelt sich meist um organische Säuren und deren Salze, Carbonate, seltener auch um anorganische Säuren und deren Salze. Der Zusatz eines Säureregulators verstärkt teils die Stabilität und Festigkeit des Lebensmittels, bewirkt eine erwünschte Ausfällung und verbessert die Wirkung von Konservierungsmitteln. Im Gegensatz zu Säuerungsmitteln werden sie nicht zur Geschmacksveränderung von Lebensmitteln benutzt. Ihre Wirkung beruht auf der Bildung eines Puffersystems im Lebensmittel, bei dem sich auf Zugabe von sauren oder basischen Stoffen der pH-Wert nicht oder nur geringfügig ändert. Beispiele sind:
- E 170: - Calciumcarbonat
- E 260-263: - Essigsäure und Acetate
- E 270: - Milchsäure
- E 296: - Äpfelsäure
- E 297: - Fumarsäure
- E 325-327: - Lactate (Milchsäure)
- E 330-333: - Citronensäure und Citrate
- E 334-337: - Weinsäure und Tartrate
- E 339-341: - Orthophosphate
- E 350-352: - Malate (Äpfelsäure)
- E 450-452: - Di-, Tri- und Polyphosphate
- E 500-504: - Carbonate (Kohlensäure)
- E 507: - Salzsäure und ChlorideE 513-517Schwefelsäure und Sulfate
- E 524-528: - Hydroxide
- E 529-530: - Oxide
- E 355-357: - Adipinsäure und Adipate
- E 574-578: - Gluconsäure und Gluconate

**Verdickungsmittel.** Verdickungsmittel sind Stoffe, die in erster Linie in der Lage sind, Wasser zu binden. Durch Entzug von ungebundenem Wasser kommt es zur Erhöhung der Viskosität. Ab einer für jedes Verdickungsmittel charakteristischen Konzentration treten zu diesem Effekt auch noch Netzwerkeffekte auf, die zu einer meist überproportionalen Erhöhung der Viskosität führen. Man spricht in diesem Fall davon, dass Moleküle miteinander 'kommunizieren', d.h. verschlaufen. Bei den meisten Verdickungsmitteln handelt es sich um lineare oder verzweigte Makromoleküle (z. B. Polysaccharide oder Proteine), die durch intermolekulare Wechselwirkungen, wie Wasserstoffbrücken, hydrophobe Wechselwirkungen oder lonenbeziehungen miteinander interagieren können. Extremfälle von Dickungsmitteln sind Schichtsilikate (Bentonite, Hectorite) oder hydratisierte SiO₂-Partikel, die als Teilchen dispergiert vorliegen und in ihrer festkörperartigen Struktur Wasser binden können bzw. aufgrund der beschriebenen Wechselwirkungen miteinander interagieren können. Beispiele sind:
- E 400: - Alginsäure
- E 401: - Natriumalginat
- E 402: - Kaliumalginat
- E 403: - Ammoniumalginat
- E 404: - Calciumalginat
- E 405: - Propylenglycolalginat
- E 406: - Agar Agar
- E 407: - Carrgeen, Furcelleran
- E 407: - Johannisbrotkernmehl
- E 412: - Guarkernmehl
- E 413: - Traganth
- E 414: - Gummi arabicum
- E 415: - Xanthan
- E 416: - Karaya (Indischer Traganth)
- E 417: - Tarakernmehl (Peruanisches Johannisbrotkernmehl)
- E 418: - Gellan
- E 440: - Pektin, Opekta
- E 440ii: - Amidiertes Pektin
- E 460: - Mikrokristalline Cellulose, Cellulosepulver
- E 461: - Methylcellulose
- E 462: - Ethylcellulose
- E 463: - Hydroxypropylcellulose
- E 465: - Methylethylcellulose
- E 466: - Carboxymethylcellulose, Natriumcarboxymethylcellulose

**Reduktionsmittel.** Die Lebensmittel können des Weiteren auch Reduktionsmittel aufweisen. Reduktionsmittel im Sinne der Erfindung sind bevorzugt in Lebensmitteln zulässige Reduktionsmittel, insbesondere die hier genannten:
- E 220: - Schwefeldioxid
- E 221: - Natriumsulfit
- E 222: - Natriumhydrogensulfit
- E 223: - Natriumdisulfit
- E 224: - Kaliumdisulfit
- E 226: - Calciumsulfit
- E 227: - Calciumhydrogensulfit
- E 228: - Kaliumhydrogensulfit
- E 300: - Ascorbinsäure
- E 301: - Natrium-L-Ascorbat
- E 302: - Calium-L-Ascorbat
- E 304: - Ascorbinsäureester
- E 306: - Tocopherol
- E 307: - Alpha-Tocopherol
- E 308: - Gamma-Tocopherol
- E 309: - Delta-Tocopherol
- E 310: - Propylgallat
- E 311: - Octylgallat
- E 312: - Dodecylgallat
- E 315: - Isoascorbinsäure
- E 316: - Natriumisoascorbat
- E 319: - tert.-Butylhydrochinon (TBHQ)
- E 320: - Butylhydroxianisol
- E 321: - Butylhydroxitoluol
- E 322: - Lecithin
- E 330: - Citronensäure
- E 512: - Zinn-II-Chlorid

### MUND- UND ZAHNPFLEGEMITTEL

Erfindungsgemäße orale Zubereitungen können auch der Mund- und Zahnreinigung und-pflege dienen. Beispiele hierfür sind Zahnpasten, Zahngele, Zahnpulver, Mundwässer und dergleichen. Unter Zahnpasten oder Zahncremes werden im allgemeinen gelförmige oder pastöse Zubereitungen aus Wasser, Verdickungsmitteln, Feuchthaltemitteln, Schleif- oder Putzkörpern, Tensiden, Süßmitteln, Aromastoffen, deodorierenden Wirkstoffen sowie Wirkstoffen gegen Mund- und Zahnerkrankungen verstanden. In die erfindungsgemäßen Zahnpasten können alle üblichen Putzkörper, wie z. B. Kreide, Dicalciumphosphat, unlösliches Natriummetaphosphat, Aluminiumsilikate, Calciumpyrophosphat, feinteilige Kunstharze, Kieselsäuren, Aluminiumoxid und Aluminiumoxidtrihydrat eingesetzt werden.

Bevorzugt geeignete Putzkörper für die erfindungsgemäßen Zahnpasten sind vor allem feinteilige Xerogelkieselsäuren, Hydrogelkieselsäuren, Fällungskieselsäuren, Aluminiumoxid-trihydrat und feinteiliges alpha -Aluminiumoxid oder Mischungen dieser Putzkörper in Mengen von 15 bis 40 Gew.-% der Zahnpasta. Als Feuchthaltemittel kommen vorwiegend niedermolekulare Polyethylenglykole, Glycerin, Sorbit oder Mischungen dieser Produkte in Mengen bis zu 50 Gew.-% in Frage. Unter den bekannten Verdickungsmitteln sind die verdickenden, feinteiligen Gelkieselsäuren und Hydrokolloide, wie z. B. Carboxymethylcellulose, Hydroxyethylcellulose, Hydroxypropylguar, Hydroxyethylstärke, Polyvinylpyrrolidon, hochmolekulares Polyethylenglykol, Pflanzengummen wie Traganth, Agar-Agar, Carragheenmoos, Gummiarabicum, Xantham-Gum und Carboxyvinylpolymere (z. B. Carbopol®-Typen) geeignet. Zusätzlich zu den Mischungen aus menthofuran und Mentholverbindungen können die Mund- und Zahnpflegemittel insbesondere oberflächenaktive Stoffe, bevorzugt anionische und nichtionische schaumstarke Tenside, wie die bereits oben genannten Stoffe, insbesondere aber Alkylethersulfat-Salze, Alkylpolyglucoside und deren Gemische.

Weitere übliche Zahnpastenzusätze sind:
- Konservierungsmittel und antimikrobielle Stoffe wie z. B. p- Hydroxybenzösäuremethyl-, -ethyl- oder -propylester, Natriumsorbat, Natriumbenzoat, Bromchlorophen, Phenylsalicylsäureester, Thymol und dergleichen;
- Antizahnsteinwirkstoffe, z. B. Organophosphate wie 1-Hydroxyethan- 1.1-diphosphonsäure, 1-Phosphonpropan-1,2,3-tricarbonsäure und andere, die z. B. aus US 3,488,419**,** DE 2224430 A1 und DE 2343196 A1 bekannt sind;
- andere karieshemmende Stoffe wie z. B. Natriumfluorid, Natriummonofluorphosphat, Zinnfluorid;
- Süssungsmittel, wie z. B. Saccharin-Natrium, Natrium-Cyclamat, Sucrose, Lactose, Maltose, Fructose oder Apartam®, (L-Aspartyl- L-phenylalanin-methylester), Stivia-extrakte oder deren süßenden Bestandteile, insbesondere Ribeaudioside;
- Zusätzliche Aromen wie z. B. Eukalyptusöl, Anisöl, Fenchelöl, Kümmelöl, Methylacetat, Zimtaldehyd, Anethol, Vanillin, Thymol sowie Mischungen dieser und anderer natürlicher und synthetischer Aromen;
- Pigmente wie z. B. Titandioxid;
- Farbstoffe;
- Puffersubstanzen wie z. B. primäre, sekundäre oder tertiäre Alkaliphosphate oder Citronensäure/Natriumcitrat;
- wundheilende und entzündungshemmende Stoffe wie z. B. Allantoin, Harnstoff, Azulen, Kamillenwirkstoffe und Acetylsalicylsäurederivate.

Eine bevorzugte Ausführung der kosmetischen Zubereitungen sind Zahnpasten in Form einer wässrigen, pastösen Dispersion, enthaltend Poliermittel, Feuchthaltemittel, Viskositätsregulatoren und gegebenenfalls weitere übliche Komponenten, sowie die Mischung aus Menthofuran und Mentholverbindungen in Mengen von 0,5 bis 2 Gew.-% enthalten.

In Mundwässern ist eine Kombination mit wässrig-alkoholischen Lösungen verschiedener Grädigkeit von ätherischen Ölen, Emulgatoren, adstringierenden und tonisierenden Drogenauszügen, zahnsteinhemmenden, antibakteriellen Zusätzen und Geschmackskorrigentien ohne weiteres möglich. Eine weitere bevorzugte Ausführung der Erfindung ist ein Mundwasser in Form einer wässrigen oder wässrig-alkoholischen Lösung enthaltend die Mischung aus Menthofuran und Mentholverbindungen in Mengen von 0,5 bis 2 Gew.-%. In Mundwässern, die vor der Anwendung verdünnt werden, können mit, entsprechend dem vorgesehenen Verdünnungsverhältnis, höheren Konzentrationen ausreichende Effekte erzielt werden.

Zur Verbesserung des Fließverhaltens können ferner Hydrotrope, wie beispielsweise Ethanol, Isopropylalkohol, oder Polyole eingesetzt werden; diese Stoffe entsprechen weitgehend den eingangs geschildern Trägern. Polyole, die hier in Betracht kommen, besitzen vorzugsweise 2 bis 15 Kohlenstoffatome und mindestens zwei Hydroxylgruppen. Die Polyole können noch weitere funktionelle Gruppen, insbesondere Aminogruppen, enthalten bzw. mit Stickstoff modifiziert sein. Typische Beispiele sind
- Glycerin;
- Alkylenglycole, wie beispielsweise Ethylenglycol, Diethylenglycol, Propylenglycol, Butylenglycol, Hexylenglycol sowie Polyethylenglycole mit einem durchschnittlichen Molekulargewicht von 100 bis 1.000 Dalton;
- technische Oligoglyceringemische mit einem Eigenkondensationsgrad von 1,5 bis 10 wie etwa technische Diglyceringemische mit einem Diglyceringehalt von 40 bis 50 Gew.-%;
- Methyolverbindungen, wie insbesondere Trimethylolethan, Trimethylolpropan, Trimethylolbutan, Pentaerythrit und Dipentaerythrit;
- Niedrigalkylglucoside, insbesondere solche mit 1 bis 8 Kohlenstoffen im Alkylrest, wie beispielsweise Methyl- und Butylglucosid;
- Zuckeralkohole mit 5 bis 12 Kohlenstoffatomen, wie beispielsweise Sorbit oder Mannit,
- Zucker mit 5 bis 12 Kohlenstoffatomen, wie beispielsweise Glucose oder Saccharose;
- Aminozucker, wie beispielsweise Glucamin;
- Dialkoholamine, wie Diethanolamin oder 2-Amino-1,3-propandiol.

Als Konservierungsmittel eignen sich beispielsweise Phenoxyethanol, Formaldehydlösung, Parabene, Pentandiol oder Sorbinsäure sowie die unter der Bezeichnung Surfacine® bekannten Silberkomplexe und die in Anlage 6, Teil A und B der Kosmetikverordnung aufgeführten weiteren Stoffklassen. Die Zubereitungen können des Weiteren auch Parfümöle enthalten, die vorzugsweise im Sinne der Erfindung verkapselt vorliegen. Geeignete Beispiele sind bereits oben genannt worden.

### KAUGUMMIS

Bei den oralen Zubereitungen kann es sich schließlich auch um Kaugummis handeln. Diese Produkte enthalten typischerweise eine wasserunlösliche und eine wasserlösliche Komponente.

Die wasserunlösliche Basis, die auch als "Gummibasis" bezeichnet wird. umfasst üblicherweise natürliche oder synthetische Elastomere, Harze, Fette und Öle, Weichmacher, Füllstoffe, Farbstoffe sowie gegebenenfalls Wachse. Der Anteil der Basis an der Gesamtzusammensetzung macht üblicherweise 5 bis 95, vorzugsweise 10 bis 50 und insbesondere 20 bis 35 Gew.-% aus. In einer typischen Ausgestaltungsform der Erfindung setzt sich die Basis aus 20 bis 60 Gew.-% synthetischen Elastomeren, 0 bis 30 Gew.-% natürlichen Elastomeren, 5 bis 55 Gew.-% Weichmachern, 4 bis 35 Gew.-% Füllstoffe und in untergeordneten Mengen Zusatzstoffe wie Farbstoffe, Antioxidantien und dergleichen zusammen, mit der Maßgabe, dass sie allenfalls in geringen Mengen wasserlöslich sind.

Als geeignete synthetische Elastomere kommen beispielsweise Polyisobutylene mit durchschnittlichen Molekulargewichten (nach GPC) von 10.000 bis 100.000 und vorzugsweise 50.000 bis 80.000, Isobutylen-Isopren-Copolymere ("Butyl Elastomere"), Styrol-Butadien-Copolymere (Styrol:Butadien-Verhältnis z.B. 1: 3 bis 3: 1), Polyvinylacetate mit durchschnittlichen Molekulargewichten (nach GPC) von 2.000 bis 90.000 und vorzugsweise 10.000 bis 65.000, Polyisoprene, Polyethylen, Vinylacetat-Vinyllaurat-Copolymere und deren Gemische. Beispiele für geeignete natürliche Elastomere sind Kautschuks wie etwa geräucherter oder flüssiger Latex oder Guayule sowie natürliche Gummistoffe wie Jelutong, Lechi caspi, Perillo, Sorva, Massaranduba balata, Massaranduba chocolate, Nispero, Rosindinba, Chicle, Gutta hang lkang sowie deren Gemische. Die Auswahl der synthetischen und natürlichen Elastomere und deren Mischungsverhältnisse richtet sich im Wesentlichen danach, ob mit den Kaugummis Blasen erzeugt werden sollen ("bubble gums") oder nicht. Vorzugsweise werden Elastomergemische eingesetzt, die Jelutong, Chicle, Sorva und Massaranduba enthalten.

In den meisten Fällen erweisen sich die Elastomere in der Verarbeitung als zu hart oder zu wenig verformbar, so dass es sich als vorteilhaft erwiesen hat, spezielle Weichmacher mitzuverwenden, die natürlich insbesondere auch alle Anforderungen an die Zulassung als Nahrungsmittelzusatzstoffe erfüllen müssen. In dieser Hinsicht kommen vor allem Ester von Harzsäuren in Betracht, beispielsweise Ester von niederen aliphatischen Alkoholen oder Polyolen mit ganz oder teilweise gehärteten, monomeren oder oligomeren Harzsäuren. Insbesondere werden für diesen Zweck die Methyl-, Glycerin-, oder Pentareythritester sowie deren Gemische eingesetzt. Alternativ kommen auch Terpenharze in Betracht, die sich von alpha-Pinen, beta-Pinen, delta-Limonen oder deren Gemischen ableiten können.

Als Füllstoffe oder Texturiermittel kommen Magnesium- oder Calciumcarbonat, gemahlener Bimsstein, Silicate, speziell Magnesium- oder Aluminiumsilicate, Tone, Aluminiumoxide. Talkum, Titandioxid, Mono-, Di- und Tricalciumphosphat sowie Cellulosepolymere.

Geeignete Emulgatoren sind Talg, gehärteter Talg, gehärtete oder teilweise gehärtete pflanzliche Öle, Kakaobutter, Partialglyceride, Lecithin, Triacetin und gesättigte oder ungesättigte Fettsäuren mit 6 bis 22 und vorzugsweise 12 bis 18 Kohlenstoffatomen sowie deren Gemische.

Als Farbstoffe und Weißungsmittel kommen beispielsweise die für die Färbung von Lebensmitteln zugelassenen FD und C-Typen, Pflanzen- und Fruchtextrakte sowie Titandioxid in Frage.

Die Basismassen können Wachse enthalten oder wachsfrei sein; Beispiele für wachsfreie Zusammensetzungen finden sich unter anderem in der Patentschrift US 5,286,500**,** auf deren Inhalt hiermit ausdrücklich Bezug genommen wird.

Zusätzlich zu der wasserunlöslichen Gummibasis enthalten Kaugummizubereitungen regelmäßig einen wasserlösliche Anteil, der beispielsweise von Softener, Süßstoffen, Füllstoffen, Geschmacksstoffen, Geschmacksverstärkern, Emulgatoren, Farbstoffen, Säuerungsmitteln, Antioxidantien und dergleichen gebildet werden, hier mit der Maßgabe, dass die Bestandteile eine wenigstens hinreichende Wasserlöslichkeit besitzen. In Abhängigkeit der Wasserlöslichkeit der speziellen Vertreter können demnach einzelne Bestandteile sowohl der wasserunlöslichen wie auch der wasserlöslichen Phase angehören. Es ist jedoch auch möglich, Kombinationen beispielsweise eines wasserlöslichen und eines wasserunlöslichen Emulgators einzusetzen, wobei sich die einzelnen Vertreter, dann in unterschiedlichen Phasen befinden. Üblicherweise macht der wasserunlösliche Anteil 5 bis 95 und vorzugsweise 20 bis 80 Gew.-% der Zubereitung aus.

Wasserlösliche Softener oder Plastifiziermittel werden den Kaugummizusammensetzungen hinzugegeben um die Kaubarkeit und das Kaugefühl zu verbessern und sind in den Mischungen typischerweise in Mengen von 0,5 bis 15 Gew.-% zugegen. Typische Beispiele sind Glycerin, Lecithin sowie wässrige Lösungen von Sorbitol, gehärteten Stärkehydrolysaten oder Kornsirup.

Als Süßstoffe kommen sowohl zuckerhaltige wie zuckerfreie Verbindungen in Frage, die in Mengen von 5 bis 95, vorzugsweise 20 bis 80 und insbesondere 30 bis 60 Gew.-% bezogen auf die Kaugummizusammensetzung eingesetzt werden. Typische Saccharid-Süssstoffe sind Sucrose, Dextrose, Maltose, Dextrin, getrockneter Invertzucker, Fructose, Levulose, Galactose, Kornsirup sowie deren Gemische. Als Zuckerersatzstoffe kommen Sorbitol, Mannitol, Xylitol, gehärtete Stärkehydrolysate, Maltitol und deren Gemische in Frage. Weiterhin kommen als Zusatzstoffe auch sogenannte HIAS ("High Intensity Articifical Sweeteners") in Betracht, wie beispielsweise Sucralose, Aspartam, Acesulfamsalze, Alitam, Saccharin und Saccharinsalze, Cyclamsäure und deren Salze, Glycyrrhizine, Dihydrochalcone, Thaumatin, Monellin und dergleichen alleine oder in Abmischungen. Besonders wirksam sind auch die hydrophoben HIAS, die Gegenstand der internationalen Patentanmeldung WO 2002 091849 A1 (Wrigleys) sowie Stevia Extrakte und deren aktiven Bestandteile, insbesondere Ribeaudiosid A sind. Die Einsatzmenge dieser Stoffe hängt in erster Linie von ihrem Leistungsvermögen ab und liegt typischerweise im Bereich von 0,02 bis 8 Gew.-%.

Insbesondere für die Herstellung kalorienarmer Kaugummis eignen sich Füllstoffe wie beispielsweise Polydextrose, Raftilose, Rafitilin, Fructooligosaccharide (NutraFlora), Palatinoseoligosaaccharide, GuarGum Hydrolysate (Sun Fiber) sowie Dextrine.

Die Auswahl an weiteren vorzugsweise im Sinne der Erfindung verkapselten Geschmacksstoffen ist praktisch unbegrenzt und für das Wesen der Erfindung unkritisch. Üblicherweise liegt der Gesamtanteil aller Geschmacksstoffe bei 0,1 bis 15 und vorzugsweise 0,2 bis 5 gew.-% bezogen auf die Kaugummizusammensetzung. Geeignete weitere Geschmacksstoffe stellen beispielsweise essentielle Öle, synthetische Aromen und dergleichen dar, wie etwa Anisöl, Sternanisöl, Kümmelöl, Eukalyptusöl, Fenchelöl, Citronenöl, Wintergrünöl, Nelkenöl, und dergleichen, wie sie auch beispielsweise in Mund- und Zahnpflegemittel Verwendung finden.

Die Kaugummis können des weiteren Hilfs- und Zusatzstoffe enthalten, die beispielsweise für die Zahnpflege, speziell zur Bekämpfung von Plaque und Gingivitis geeignet sind, wie z.B. Chlorhexidin, CPC oder Trichlosan. Weiter können pH-Regulatoren (z.B. Puffer oder Harnstoff), Wirkstoffe gegen Karies (z.B. Phosphate oder Fluoride), biogene Wirkstoffe (Antikörper, Enzyme, Koffein, Pflanzenextrakte) enthalten sein, solange diese Stoffe für Nahrungsmittel zugelassen sind und nicht in unerwünschter Weise miteinander in Wechselwirkung treten.

Offenbart wir zudem die Verwendung sowohl von hydrolysierbaren als auch nichthydrolysierbaren Tanninen als Vernetzer bzw. Härtungsmittel bei der Herstellung von Wirkstoffkapseln mit einer Hülle aus Proteinen und/oder Polysacchariden.

Ein letzter Gegenstand der Erfindung betrifft die Verwendung der erfindungsgemäßen Wirkstoffkapseln zur Herstellung von Zubereitungen zur oralen Aufnahme.

### BEISPIELE

### HERSTELLBEISPIELE

### BEISPIEL 1

### Herstellung eines verkapselten Zitrusaromas

In einem Rührkessel wurden 350 kg Wasser (Trinkwasserqualität) vorgelegt und bei etwa 40 % der Leistung des Rührwerks auf 80 °C aufgeheizt. Anschließend wurde 2,4 kg Gummi Arabicum (Typ Seyal), 1,8 kg Carboxymethylcellulose (Ceroga 4510C, Fa. Röper) sowie 11.8 kg Rindergelatine (240 Bloom) zugegeben und die Mischung 45 min gerührt. Anschließend wurde auf 55 °C abgekühlt und 115 kg Zitrusaroma zugegeben, wobei sich die Temperatur auf ca. 42 °C abkühlte. Durch Zugabe von 1,2 kg Essigsäure wurde der pH-Wert auf 4,2 eingestellt und zur Schaumvermeidung 0,4 kg eines handelsüblichen Silikonentschäumers hinzugefügt. Die Rührgeschwindigkeit wurde auf 75 % erhöht und der Tankinhalt innerhalb von 2h langsam von 40 °C auf etwa 10 °C abgekühlt. Bei Erreichen von 20 °C wurde die Rührleistung auf 40 % abgesenkt. Bei 10 °C wurde schließlich eine Zubereitung von 1,4 kg Tannin in 5 I Wasser langsam zugegeben. Die Kühlung wurde ausgestellt und der Ansatz weitere 8 h gerührt. Die gebildeten Kapseln wiesen einen D50 von etwa 50 µm auf.

### BEISPIEL 2

### Sprühtrocknung der Kapseln

484 kg der wässrigen Kapseldispersion aus Beispiel 1 wurden in einem Kessel unter Rühren auf 40 °C erhitzt und langsam mit 115 kg Maltodextrin auf Weizenbasis vermischt. Anschließend wurde die Mischung über einen Sprühturm gegeben. Die Eintrittstemperatur betrug 180 bis 185 °C, die Austrittstemperatur 72 bis 78 °C.

### BEISPIEL 3

### Kontrollierte Freisetzung der Wirkstoffe aus den Kapseln

Die erfindungsgemäßen Kapseln sind nicht wasserlöslich, was dazu führt, dass sie in wässriger Umgebung den enthaltenen Wirkstoff nur verzögert freigeben. Zu diesem Zweck wurden die Beispiele 1 und 2 wiederholt, jedoch der Aromastoff gewichtsgleich gegen Neutral ausgetauscht, das mit einem blauen Farbstoff versetzt worden war. Anschließend wurden die Kapseln in farblosem Neutralöl dispergiert und die Freisetzung des Farbstoffs aus den Kapseln, d.h. die Anfärbung des Neutralöls über die Zeit mittel Lab-Farbmesssystem verfolgt. Die Ergebnisse sind in **Tabelle 1** zusammengefasst.

**Tabelle 1**

| Kontrollierte Freisetzung von blauem Farbstoff | |
|---|---|
| **Zeit [min]** | **Relative Farbintensität [%]** |
| 1 | 29 |
| 2 | 30 |
| 3 | 31 |
| 4 | 35 |
| 5 | 37 |
| 15 | 41 |
| 30 | 43 |
| 45 | 52 |
| 60 | 55 |
| 120 | 67 |
| 180 | 80 |
| 1.200 | 95 |

### BEISPIEL 4

### Farbfreisetzung bei Scherung und höheren Temperaturen

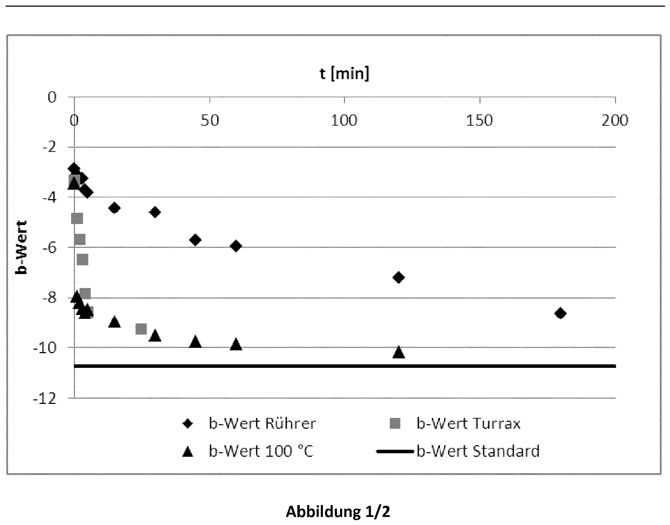

Um zu belegen, welchen Einfluss eine Erhitzung oder eine starke Scherung mittels Ultra-Turrax auf das Freisetzungsverhalten haben, wurden die Kapseln mit den gefärbten Öl abermals in Neutralöl dispergiert. Eine Probe wurde nur gerührt und die Blaufärbung des Dispersionsmediums gemessen. Die zweite Probe wurde mit einem Ultra-Turrax stark geschert und die dritte Probe wurde bei einer Temperatur von 100°C gehalten. Die unterschiedlichen Freisetzungsverläufe sind anhand des "blau"-Wertes in **Abbildung 1** wiedergegeben.

### Beispiel 5

### Aromafreisetzung in einer Kaugummiformulierung

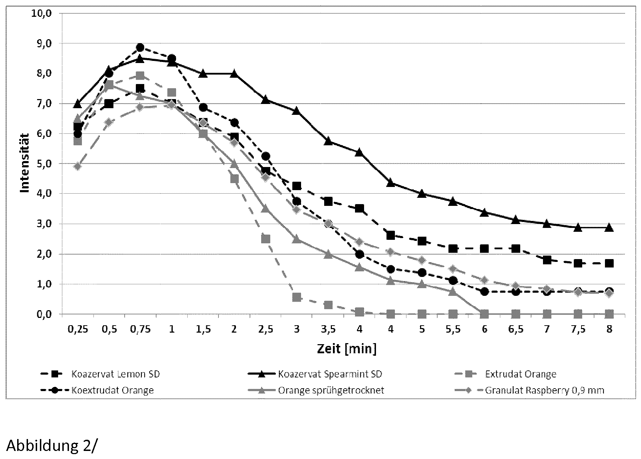

Eine Standardkaugummi-Formulierung wurde mit gleichen Mengen unterschiedlicher Kapseln mit Zitronen, orangen und Spearmint-Aroma versetzt und die Freisetzung der Aromen über die Zeit verfolgt **(****Abbildung 2****).** Es ist deutlich zu erkennen, dass die neuen sprühgetrockneten Koazervate das Aroma deutlich verlangsamt freisetzen und somit die für Kaugummi angestrebte lang dauernde Aromafreisetzung und somit auch Aromawahrnehmung bewirken.

### ANWENDUNGSBEISPIELE

### ANWENDUNGSBEISPIEL 1

In der folgenden **Tabelle 2** sind zwei Formulierungen für ein Eistee-Getränk (Schwarztee) angegeben. Schwarztee-Extrakt wurde in Wasser gelöst und zusammen mit Zucker, einer Aromazubereitung sowie dem Aronia-Extrakt A in einem Becherglas verrührt.

**Tabelle 2**

| Anwendungsformulierungen | | |
|---|---|---|
| **Inhaltsstoff** | **Einsatz in Gew.-%** | |
| | **A** | **B** |
| Schwarztee-Extrakt | 1,4 | 1,4 |
| Zucker | 7 | 7 |
| Zitronensäure (kristallin) | 1,2 | 1,2 |
| Ascorbinsäure | 0,2 | 0,2 |
| **Mikrokapseln nach Beispiel 2 mit Pfirsicharoma** | 1.0 | 1,5 |
| Wasser | Ad 100 | |

### ANWENDUNGSBEISPIEL 2

In der folgenden **Tabelle 3** sind zwei weitere Formulierungen für ein Eistee-Getränk (Grüntee, zuckerreduziert) angegeben. Grüntee-Extrakt wurde in Wasser gelöst und zusammen mit Zucker, sowie dem Süßstoff Saccharin bzw. Rebaudiosid A, einer Aromazubereitung sowie dem Aronia-Extrakt A in einem Becherglas verrührt.

**Tabelle 3**

| Anwendungsformulierungen | | |
|---|---|---|
| **Inhaltsstoff** | **Einsatz in Gew.-%** | |
| | **A** | **B** |
| Grüntee-Extrakt | 1,4 | 1,4 |
| Zucker | 3,45 | 3,45 |
| Süßstoff Saccharin | 0,1 | --- |
| Süßstoff Rebaudiosid A | --- | 0,02 |
| Zitronensäure (kristallin) | 1,2 | 1,2 |
| Ascorbinsäure | 0,2 | 0,2 |
| **Mikrokapseln nach Beispiel 2 mit Zitrusaroma** | 1,0 | 1,5 |
| Wasser | Ad 100 | |

### ANWENDUNGSBEISPIEL 3

In der folgenden **Tabelle 4** sind zwei weitere Formulierungen für ein Eistee-Getränk (Schwarztee, zuckerfrei) angegeben. Schwarztee-Extrakt wurde in Wasser gelöst und zusammen mit dem Süßstoff Saccharin, einer Aromazubereitung, sowie dem Aronia-Extrakt A in einem Becherglas verrührt.

**Tabelle 4**

| Anwendungsformulierungen | | |
|---|---|---|
| **Inhaltsstoff** | **Einsatz in Gew.-%** | |
| | **A** | **B** |
| Schwarztee-Extrakt | 1,4 | 1,4 |
| Saccharin | 0,035 | 0,035 |
| Zitronensäure (kristallin) | 1,2 | 1,2 |
| Ascorbinsäure | 0,2 | 0,2 |
| **Mikrokapseln nach Beispiel 2 mit Zitrusaroma** | 1,0 | 1,5 |
| Wasser | Ad 100 | |

### ANWENDUNGSBEISPIEL 4

In der folgenden **Tabelle 5** sind Formulierungen für ein Soja-Getränk angegeben. Der Aronia-Extrakt wurde zu einer Sojamilch aus einem lokalen Supermarkt hinzugefügt. Die Mischung wurde zusammen mit dem Milcharoma im Becherglas verrührt.

**Tabelle 5**

| Anwendungsformulierungen | | | | |
|---|---|---|---|---|
| **Inhaltsstoff** | **Einsatz in Gew.-%** | | | |
| | **A** | **B** | **C** | **D** |
| Sojamilch (nicht aromatisiert, ungesüßt) | Ad 100 | | | |
| Saccharose | 3,0 | - | 1,5 | 2,0 |
| Sucralose | - | 0,025 | 0,01 | - |
| Na-Saccharin | - | - | 0,01 | - |
| Emulgum | 0,1 | 0,1 | - | 0,1 |
| **Mikrokapseln nach Beispiel 2 mit Vanillearoma** | 0,5 | 0,75 | 1,0 | 1,5 |
| Hesperetin, 5 %ig in Ethanol | - | - | - | 0,1 |

### ANWENDUNGSBEISPIEL 5

In der folgenden **Tabelle 6** ist eine Formulierung für ein Soja-Getränk in Kombination mit γ-Aminobuttersäure angegeben. Die γ-Aminobuttersäure wurde in Wasser vorgelöst und zusammen mit dem Aronia-Extrakt A zu einer Sojamilch aus einem lokalen Supermarkt hinzugefügt. Die resultierende Mischung wurde zusammen mit dem Milcharoma im Becherglas verrührt.

**Tabelle 6**

| Anwendungsformulierungen | |
|---|---|
| **Inhaltsstoff** | **Einsatz in Gew.-%** |
| Sojamilch (aus lokalem Supermarkt) | 98,3 |
| **Mikrokapseln nach Beispiel 2 mit Milcharoma** | 1,5 |
| γ-Aminobuttersäure, 1%ig in Wasser | 0,1 |

### ANWENDUNGSBEISPIEL 6

In der folgenden **Tabelle 7** ist eine Formulierung für einen Grapefruitsaft angegeben. Die Verbindungen (1) und (2) wurden in Ethanol vorgelöst und zu einem Grapefruitsaft aus einem lokalen Supermarkt hinzugefügt. Die resultierende Mischung wurde in einem Becherglas durch Rühren homogenisiert.

**Tabelle 7**

| Anwendungsformulierungen | |
|---|---|
| **Inhaltsstoff** | **Einsatz in Gew.-%** |
| Grapefruitsaft (aus lokalem Supermarkt) | 98,5 |
| **Mikrokapseln nach Beispiel 2 mit Zitrusaroma** | 1,5 |

### ANWENDUNGSBEISPIEL 7

In der folgenden **Tabelle 8** sind zwei Formulierungen für bittere Schokolade angegeben. Dazu wurde eine bittere Schokolade aus folgenden Rohstoffen hergestellt und anschließend in rechteckigen Formen ausgegossen:

**Tabelle 8**

| Anwendungsformulierungen | | |
|---|---|---|
| **Inhaltsstoff** | **Einsatz in Gew.-%** | |
| | **A** | **B** |
| Kakaomasse | Ad 100 | Ad 100 |
| Kakaobutter | 11,70 | 11,70 |
| Zucker | 29,50 | 29,50 |
| Magermilch | 3,00 | 3,00 |
| Lecithin | 0,2 | 0,2 |
| Vanillin | 0,035 | 0,035 |
| **Mikrokapseln nach Beispiel 2 mit Chiliaroma** | 1,0 | 1,5 |

### ANWENDUNGSBEISPIEL 8

In der folgenden **Tabelle 10** ist eine Formulierung für ein Kaugummi wiedergegeben. Die Teile A bis D wurden gemischt und intensiv geknetet. Die Rohmasse kann z.B. in Form von dünnen Streifen zu verzehrfertigen Kaugummis verarbeitet werden.

**Tabelle 9**

| Anwendungsformulierungen | | |
|---|---|---|
| **Teil** | **Inhaltsstoff** | **Einsatz in Gew.-%** |
| A | Kaugummibase, Company "Jagum T" | 30,00 |
| B | Sorbit, pulverisiert | 39,00 |
| | Isomalt® (Palatinit GmbH) | 9,50 |
| | Xylit | 2,00 |
| | Mannit | 3,00 |
| | Aspartam® | 0,10 |
| | Acesulfam® K | 0,10 |
| | Emulgum® (Colloides Naturels, Inc.) | 0,30 |
| C | Sorbitol, 70%ig in Wasser | 14,00 |
| | Glycerin | 1,00 |
| D | **Mikrokapseln nach Beispiel 2 mit Sparemintaroma** | 1,00 |

### ANWENDUNGSBEISPIEL 9

In der folgenden **Tabelle 11** ist eine Formulierung für eine Zahnpasta wiedergegeben. Die Inhaltsstoffe der Teile A und B wurden jeweils für sich vorgemischt und zusammen unter Vakuum bei 25 - 30°C 30 min gut verrührt. Teil C wurde vorgemischt und zu A und B gegeben; D wurde hinzugefügt und die Mischung unter Vakuum bei 25 - 30°C für 30 min gut verrührt. Nach Entspannung war die Zahnpasta fertig und konnte abgefüllt werden.

**Tabelle 10y**

| Anwendungsformulierungen | | |
|---|---|---|
| **Teil** | **Inhaltsstoff** | **Einsatz in Gew.-%** |
| A | demineralisiertes Wasser | 21,50 |
| | Sorbitol (70%) | 45,00 |
| | Solbrol® M, Natriumsalz (Bayer AG, p-Hydroxybenzoesäurealkylester) | 0,15 |
| | Trinatriumphosphat | 0,10 |
| | Saccharin, 450fach | 0,20 |
| | Natriummonofluorphosphat | 1,12 |
| | Polyethylenglycol 1500 | 5,00 |
| B | Sident 9 (abrasives Siliciumdioxid) | 10,00 |
| | Sident 22 S (verdickendes Siliciumdioxid) | 8,00 |
| | Natriumcarboxymethylcellulose | 0,90 |
| | Titandioxid | 0,50 |
| C | demineralisiertes Wasser | 4,53 |
| | Natriumlaurylsulfat | 1,50 |
| D | **Mikrokapseln nach Beispiel 2 mit Minzaroma** | 1,50 |

## Patentansprüche

1. Wirkstoffkapseln, dadurch erhältlich, dass man unter Eintrag von mechanischer und thermischer Energie
(a) Proteine, Polysaccharide und Cellulosederivate in Wasser löst bzw. dispergiert,
(b) die zu verkapselnden Wirkstoffe zugibt,
(c) die so erhaltenen Koazervate unter Zugabe von Tanninen aushärtet und gegebenenfalls
(d) anschließend einer Sprühtrocknung unterwirft,
mit der Maßgabe, dass die Kapseln frei von Formaldehyd und Glutaraldehyd sind.

2. Verfahren zur Herstellung von Wirkstoffkapseln, bei dem man unter Eintrag von mechanischer und thermischer Energie
(a) Proteine, Polysaccharide und Cellulosederivate in Wasser löst bzw. dispergiert,
(b) die zu verkapselnden Wirkstoffe zugibt,
(c) die so erhaltenen Koazervate unter Zugabe von Tanninen aushärtet und gegebenenfalls
(d) anschließend einer Sprühtrocknung unterwirft,
mit der Maßgabe, dass die Kapseln frei von Formaldehyd und Glutaraldehyd sind

3. Verfahren nach Anspruch 2, **dadurch gekennzeichnet, dass** man als Proteine (Komponente a1) Gelatinen einsetzt, die eine Gelfestigkeit von 100 bis 300 Bloom aufweisen.

4. Verfahren nach den Ansprüchen 2 und/oder 3, **dadurch gekennzeichnet, dass** man Polysaccharide (Komponente a2) einsetzt, die ausgewählt sind aus der Gruppe, die gebildet wird von Pektinen, Xanthanen, Gummi arabicum und Agarosen.

5. Verfahren nach mindestens einem der Ansprüche 2 bis 4, **dadurch gekennzeichnet, dass** man als Cellulosederivate (Komponente a3) Celluloseether oder Celluloseester einsetzt, die einen Substitutionsgrad oberhalb von 80 % aufweisen.

6. Verfahren nach mindestens einem der Ansprüche 2 bis 5, **dadurch gekennzeichnet, dass** man als Wirkstoffe (Komponente b) Aromen oder Parfümöle einsetzt.

7. Verfahren nach mindestens einem der Ansprüche 2 bis 6, **dadurch gekennzeichnet, dass** man die Komponenten (a1), (b1) und (c1) im Gewichtsverhältnis von etwa 10:(1 bis 5):(0,5 bis 2) einsetzt.

8. Verfahren nach mindestens einem der Ansprüche 2 bis 7, **dadurch gekennzeichnet, dass** man die Komponenten (a1+a2+a3) und (b) im Gewichtsverhältnis von etwa (5 bis 15):1 einsetzt.

9. Verfahren nach mindestens einem der Ansprüche 2 bis 8, **dadurch gekennzeichnet, dass** man die Koazervation bei Temperaturen im Bereich von etwa 20 bis etwa 90 °C durchführt.

10. Verfahren nach mindestens einem der Ansprüche 2 bis 9, **dadurch gekennzeichnet, dass** man zur Härtung Tannine in Mengen von etwa 0,1 bis etwa 2 Gew.-% - bezogen auf die Koazervate - zusetzt.

11. Verfahren nach mindestens einem der Ansprüche 2 bis 10, **dadurch gekennzeichnet, dass** man die gehärteten Kapseln in Gegenwart von Polysacchariden sprühtrocknet.

12. Zubereitungen zur oralen Aufnahme, enthaltend die Wirkstoffkapseln nach Anspruch 1.

13. Verwendung von Wirkstoffkapseln nach Anspruch 1 zur Herstellung von Zubereitungen zur oralen Aufnahme.

## Claims

1. Active ingredient capsules obtainable by, with the input of mechanical and thermal energy,
(a) dissolving and/or dispersing proteins, polysaccharides and cellulose derivatives in water,
(b) adding the active ingredients to be encapsulated,
(c) hardening the resulting coacervates with the addition of tannins and optionally
(d) then subjecting them to a spray drying,
on condition that the capsules are free of formaldehyde and glutaraldehyde.

2. Process for producing active ingredient capsules, in which, with the input of mechanical and thermal energy,
(a) proteins, polysaccharides and cellulose derivatives are dissolved and/or dispersed in water,
(b) the active ingredients to be encapsulated are added,
(c) the resulting coacervates are hardened with the addition of tannins and optionally
(d) then subjected to a spray drying
on condition that the capsules are free of formaldehyde and glutaraldehyde.

3. Process according to Claim 2, **characterized in that** the proteins (component a1) used are gelatines which have a gel strength of from 100 to 300 Bloom.

4. Process according to Claim 2 and/or 3, **characterized in that** polysaccharides (component a2) are used which are selected from the group which is formed by pectins, xanthans, gum arabic and agaroses.

5. Process according to at least one of Claims 2 to 4, **characterized in that** the cellulose derivatives (component a3) used encompass cellulose ethers or cellulose esters which have a degree of substitution above 80%.

6. Process according to at least one of Claims 2 to 5, **characterized in that** the active ingredients (component b) used are aromas or perfume oils.

7. Process according to at least one of Claims 2 to 6, **characterized in that** the components (a1), (b1) and (c1) are used in the weight ratio of about 10:(1 to 5):(0.5 to 2).

8. Process according to at least one of Claims 2 to 7, **characterized in that** the components (a1+a2+a3) and (b) are used in the weight ratio of about (5 to 15):1.

9. Process according to at least one of Claims 2 to 8, **characterized in that** the coacervation is carried out at temperatures in the range from about 20 to about 90°C.

10. Process according to at least one of Claims 2 to 9, **characterized in that,** for the hardening, tannins are added in amounts of from about 0.1 to about 2% by weight - based on the coacervates.

11. Process according to at least one of Claims 2 to 10, **characterized in that** the hardened capsules are spray-dried in the presence of polysaccharides.

12. Preparations for oral administration, comprising the active ingredient capsules according to Claim 1.

13. Use of active ingredient capsules according to Claim 1 for producing preparations for oral administration.

## Revendications

1. Capsules d'agents actifs, pouvant être obtenues en ce qu'avec apport d'énergie mécanique et thermique,
(a) des protéines, des polysaccharides et des dérivés de cellulose sont dissous ou dispersés dans de l'eau,
(b) les agents actifs à encapsuler sont ajoutés,
(c) les coacervats ainsi obtenus sont durcis avec ajout de tannins et éventuellement
(d) ensuite soumis à un séchage par pulvérisation,
à condition que les capsules soient exemptes de formaldéhyde et de glutaraldéhyde.

2. Procédé de fabrication de capsules d'agents actifs, selon lequel, avec apport d'énergie mécanique et thermique,
(a) des protéines, des polysaccharides et des dérivés de cellulose sont dissous ou dispersés dans de l'eau,
(b) les agents actifs à encapsuler sont ajoutés,
(c) les coacervats ainsi obtenus sont durcis avec ajout de tannins et éventuellement
(d) ensuite soumis à un séchage par pulvérisation,
à condition que les capsules soient exemptes de formaldéhyde et de glutaraldéhyde.

3. Procédé selon la revendication 2, **caractérisé en ce que** des gélatines qui présentent une résistance de gel de 100 à 300 Bloom sont utilisées en tant que protéines (composant a1).

4. Procédé selon les revendications 2 et/ou 3, **caractérisé en ce que** des polysaccharides (composant a2) qui sont choisis dans le groupe formé par les pectines, les xanthanes, la gomme arabique et les agaroses sont utilisés.

5. Procédé selon au moins l'une quelconque des revendications 2 à 4, **caractérisé en ce que** des éthers de cellulose ou des esters de cellulose qui présentent un degré de substitution supérieur à 80 % sont utilisés en tant que dérivés de cellulose (composant a3).

6. Procédé selon au moins l'une quelconque des revendications 2 à 5, **caractérisé en ce que** des arômes ou des huiles parfumées sont utilisés en tant qu'agents actifs (composant b).

7. Procédé selon au moins l'une quelconque des revendications 2 à 6, **caractérisé en ce que** les composants (a1), (b1) et (c1) sont utilisés en un rapport en poids d'environ 10:(1 à 5):(0,5 à 2).

8. Procédé selon au moins l'une quelconque des revendications 2 à 7, **caractérisé en ce que** les composants (a1+a2+a3) et (b) sont utilisés en un rapport en poids d'environ (5 à 15):1.

9. Procédé selon au moins l'une quelconque des revendications 2 à 8, **caractérisé en ce que** la coacervation est réalisée à des températures dans la plage allant d'environ 20 à environ 90 °C.

10. Procédé selon au moins l'une quelconque des revendications 2 à 9, **caractérisé en ce que** des tannins sont ajoutés en des quantités d'environ 0,1 à environ 2 % en poids, par rapport aux coacervats, pour le durcissement.

11. Procédé selon au moins l'une quelconque des revendications 2 à 10, **caractérisé en ce que** les capsules durcies sont séchées par pulvérisation en présence de polysaccharides.

12. Préparations pour l'administration orale, contenant les capsules d'agents actifs selon la revendication 1.

13. Utilisation de capsules d'agents actifs selon la revendication 1 pour la fabrication de préparations pour l'administration orale.
